(19) Europäisches Patentamt
European Patent Office
Office européen des brevets

(11) **EP 4 371 966 A1**

(12) **EUROPEAN PATENT APPLICATION**

(43) Date of publication:
**22.05.2024 Bulletin 2024/21**

(21) Application number: **23206599.5**

(22) Date of filing: **30.10.2023**

(51) International Patent Classification (IPC):
*C04B 41/00* (2006.01)  *C04B 41/52* (2006.01)
*C04B 41/89* (2006.01)  *C04B 35/486* (2006.01)
*C04B 35/632* (2006.01)  *C04B 35/634* (2006.01)
*C04B 35/638* (2006.01)  *A61C 13/00* (2006.01)
*A61C 13/083* (2006.01)  *A61K 6/818* (2020.01)
*A61K 6/822* (2020.01)  *C04B 111/00* (2006.01)
*C04B 111/82* (2006.01)

(52) Cooperative Patent Classification (CPC):
(C-Sets available)
**C04B 41/009; A61C 13/0022; A61C 13/082;
A61C 13/083; A61K 6/818; A61K 6/822;
A61K 6/824; C04B 35/486; C04B 35/632;
C04B 35/63488; C04B 35/638; C04B 41/0072;
C04B 41/52; C04B 41/89;** C04B 2111/00405;

(Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **28.10.2022 JP 2022173777**

(71) Applicant: **Shofu Inc.
Kyoto-shi, Kyoto 605-0983 (JP)**

(72) Inventors:
• **NONAKA, Kazumichi
Kyoto, 605-0983 (JP)**
• **TAKAHASHI, Shuhei
Kyoto, 605-0983 (JP)**

(74) Representative: **Eisenführ Speiser
Patentanwälte Rechtsanwälte PartGmbB
Gollierstraße 4
80339 München (DE)**

(54) **ZIRCONIA MILL BLANK AND PREPARING METHOD THEREOF**

(57) To provide a preparing method of a zirconia mill blank for dental cutting and machining having any one or more of translucency gradation, color gradation and strength gradation after perfect sintering by impregnating a zirconia calcined body with a plurality of impregnating solutions, which can suppress deterioration of the fitting property of a dental prosthesis device prepared from the zirconia mill blank for dental cutting and machining and a zirconia mill blank for dental cutting and machining having any one or more of translucency gradation, color gradation and strength gradation after perfect sintering and suppressing deterioration of the fitting property of a dental prosthesis device prepared from the zirconia mill blank for dental cutting and machining.

To porovide a zirconia mill blank for dental cutting and machining of the present invention is a preparing method of a zirconia mill blank for dental cutting and machining having any one or more of translucency gradation, color gradation and strength gradation after perfect sintering, comprising a step of impregnating a zirconia calcined body with at least two types of impregnating solutions, wherein at least one of the impregnating liquids contains a zirconium component, and in a case in which the total of metal ion concentrations (mass%) contained in each impregnating liquid is defined as $C_{total}$, the difference $\Delta C_{total}$ which is a difference between $C_{total}$ of each impregnating liquid is 5.0 or less.

**(Cont. next page)**

(52) Cooperative Patent Classification (CPC): (Cont.)
C04B 2111/00836; C04B 2111/82;
C04B 2235/3224; C04B 2235/3225;
C04B 2235/3272; C04B 2235/3279;
C04B 2235/443; C04B 2235/449;
C04B 2235/616; C04B 2235/6567;
C04B 2235/75; C04B 2235/77; C04B 2235/775;
C04B 2235/9615; C04B 2235/9653;
C04B 2235/9661

C-Sets
**C04B 41/009, C04B 35/48, C04B 38/00;**
**C04B 41/52, C04B 41/4539, C04B 41/5009,**
**C04B 2103/0014, C04B 2103/0013,**
**C04B 41/5042;**
**C04B 41/52, C04B 41/4539, C04B 41/5009,**
**C04B 2103/0021**

**Description**

BACKGROUND OF THE INVENTION

Field of the Invention

The present invention relates to a zirconia mill blank for dental cutting and machining and a preparing method thereof.

Description of the Related Art

In recent years, techniques to prepare a prosthesis device by the cutting and machining which uses the dental CAD/CAM system has been spread rapidly and therefore it has been becoming possible to easily prepare prosthetic devices by cutting and machining the mill blanks which are made of ceramic materials such as a zirconia, an alumina and a lithium disilicate glass, or resin materials such as an acrylic resin and a hybrid resin.
In particular, the zirconia has been clinically applied in various cases because of its high strength. On the other hand, the perfect sintered zirconia (hereinafter, also referred to as "zirconia perfect sintered body") has very high hardness and therefore cannot be cut and machined using a dental CAD / CAM system. Thus, a zirconia which is not fully sintered but is calcined at a low firing temperature to adjust to a hardness that enables to cut has been used as a zirconia mill blank for dental cutting and machining.
A general zirconia mill blank for dental cutting and machining is prepared by molding a zirconia powder by press molding or the like and then calcining at 800 to 1200 °C.
In recent years, the technique of coating and/or impregnating a zirconia mill blank for dental cutting and machining with a solution containing stabilizers or colorants has been widely used to adjust color tone and translucency.
Japanese Unexamined Patent Application Publication No. 2018-15364 discloses a method of obtaining translucency gradation and/or color gradation by impregnating multiple solutions containing stabilizers and/or colorants into a zirconia calcined body. The zirconia perfect sintered body prepared by this method has natural translucency gradation and natural color tone gradation.

SUMMARY OF THE INVENTION

Technical Problem

However, in this method, when a metal ion content of each solution differs each other, there has been a problem that the shrinkage rate differs depending on the part of the zirconia calcined body and therefore fitting property after sintering deteriorates.
An object of the present invention is to provide a preparing method of a zirconia mill blank for dental cutting and machining having any one or more of translucency gradation, color gradation and strength gradation after perfect sintering by impregnating a zirconia calcined body with a plurality of impregnating solutions, which can suppress deterioration of the fitting property of a dental prosthesis device prepared from the zirconia mill blank for dental cutting and machining and a zirconia mill blank for dental cutting and machining having any one or more of translucency gradation, color gradation and strength gradation after perfect sintering and suppressing deterioration of the fitting property of a dental prosthesis device prepared from the zirconia mill blank for dental cutting and machining.

Solution to Problem

The present inventors made a study of a preparing method of a zirconia mill blank for dental cutting and machining having any one or more of translucency gradation, color gradation and strength gradation after perfect sintering by impregnating a zirconia calcined body with a plurality of impregnating solutions, which can suppress deterioration of the fitting property of a dental prosthesis device prepared from the zirconia mill blank for dental cutting and machining. As a result, the present inventors have found that it is particularly important that at least two types of impregnating solutions are impregnated into the zirconia calcined body, at least one of the impregnating liquids contains a zirconium component, and in a case in which the total of metal ion concentrations (mass%) contained in each impregnating liquid is defined as $C_{total}$, the difference $\Delta C_{total}$ which is a difference between $C_{total}$ of each impregnating liquid is 5.0 or less, for preventing the deterioration of the fitting property of a dental prosthesis device without deteriorating the properties of the zirconia perfect sintered body. The details of the invention are described below.
The preparing method of a zirconia mill blank for dental cutting and machining of the present invention is a preparing method of a zirconia mill blank for dental cutting and machining having any one or more of translucency gradation, color gradation and strength gradation after perfect sintering, comprising

> a step of impregnating a zirconia calcined body with at least two types of impregnating solutions, wherein
> at least one of the impregnating liquids contains a zirconium component, and
> in a case in which the total of metal ion concentrations (mass%) contained in each impregnating liquid is defined as $C_{total}$,
> the difference $\Delta C_{total}$ which is a difference between $C_{total}$ of each impregnating liquid is 5.0 or less.

In the present invention, the preparing method may further comprise a step of drying the zirconia calcined body impregnated with the impregnating solution.

In the present invention, the preparing method may further comprise a step of degreasing the zirconia calcined body impregnated with the impregnating solution.

In the present invention, at least one of the impregnating solution may contain a transition metal ion.

In the present invention, at least one of the impregnating solution may contain a rare earth metal ion.

In the present invention, at least one of the impregnating solution may contain any one or aluminum ion, gallium ion, and indium ion.

In the present invention, the zirconium component in the impregnating solution may be any one or more of zirconium oxychloride, zirconium oxyacetate, and zirconyl nitrate.

In the present invention, at least one of the impregnating solution may contain urea.

In the present invention, at least one of the impregnating solution may contain glycol.

In the present invention, at least one of the impregnating solution may contain a hydroxy acid.

In the present invention, the hydroxy acid may be any one or more of citric acid, malic acid and lactic acid.

The present invention also provides zirconia mill blank for dental cutting and machining wherein, in a case in which, in bewteen two opposing surfaces, a section from one end to 50% of a distance between the two opposing surfaces is defined as section A, a section from other end to 50% of the distance between the two opposing surfaces is defined as section B, the section A contains a region A in which a compound of a stabilizer element is supported in a pore, the section B contains a region B in which a zirconium compound is supported in a pore, and a concentration of a metal ion in the region A and a concentration of a metal ion in the region B are different and/or a type of a metal ion in the region A and a type of a metal ion in the region B are different.

In the present invention, a stabilizer concentration in the zirconium compound may be lower than a stabilizer concentration in the zirconia mill blank for dental cutting and machining which constitutes a base material.

In the present invention, the stabilizer element may be a rare earth metal.

In the present invention, the rare earth metal may be yttrium.

In the present invention, a compound of a coloring element may be supported in the pore of at least one of the region A and the region B.

In the present invention, the coloring element may be a rare earth metal.

In the present invention, the coloring element may be a transition metal.

In the present invention, at least one of an aluminum compound, a gallium compound and an indium compound may be supported in the pore of at least one of the region A and the region B.

In the present invention, at least one of a niobium compound and a tantalum compound may be supported in the pore of at least one of the region A and the region B.

In the present invention, a difference in relative density between the region A and the region B may be less than 0.008.

Advantageous Effects of Invention

According to the present invention, a preparing method of a zirconia mill blank for dental cutting and machining having any one or more of translucency gradation, color gradation and strength gradation after perfect sintering by impregnating a zirconia calcined body with a plurality of impregnating solutions, which can suppress deterioration of the fitting property of a dental prosthesis device prepared from the zirconia mill blank for dental cutting and machining and a zirconia mill blank for dental cutting and machining having any one or more of translucency gradation, color gradation and strength gradation after perfect sintering and suppressing deterioration of the fitting property of a dental prosthesis device prepared from the zirconia mill blank for dental cutting and machining are provided.

DETAILED DESCRIPTION OF THE PREFERRED EMBODIMENTS

Hereinafter, specific embodiments of the requirments of the present invention will be described in detail. The present invention relates to a preparing method of a zirconia mill blank for dental cutting and machining having any one or more of translucency gradation, color gradation and strength gradation after perfect sintering, comprising a step of impregnating a zirconia calcined body with at least two types of impregnating solutions, wherein at least one of the impregnating liquids contains a zirconium component, and in a case in which the total of metal ion concentrations (mass%) contained in each impregnating liquid is defined as $C_{total}$, the difference $\Delta C_{total}$ which is a difference between $C_{total}$ of each impregnating liquid is 5.0 or less.

The $\Delta C_{total}$ is preferably 4.0 or less and is more preferably 2.0 or less. When the $\Delta C_{total}$ exceeds 5.0, there is a tendency that fitting property in the prepared dental prosthesis device deteriorates. When the $\Delta C_{total}$ is 4.0 or less, it is possibelt to fit a prosthesis device to the abutment tooth with only slight modification after sintering. When the $\Delta C_{total}$ is 2.0 or less, it is possibelt to fit a prosthesis device to the abutment tooth with little modification after sintering.

As the metal ion of the present invention, any metal ion can be used without particular limitation as long as it can be dissolved in a solvent. Specific examples include an yttrium ion which may be used a stabilizer for zirconia, praseodymium ion, neodymium ion, terbium ion, erbium ion and iron ion which are used as a coloring component, and aluminum ion, gallium ion and indium ion which are used for the purpose of controlling sinterability, niobium ion and tantalum ion which are used for the purpose of improving fracture toughness.

In the present invention, stabilizer refers to an oxide and/or a metal ion in oxide that are solid-solved in zirconia crystal and stabilize the tetragonal or cubic crystal of the zirconia.

In the present invention, colorant refers to an oxide and/or a metal ion in oxide that color zirconia after sintering.

Examples of the stabilizer include oxides or metal ions that color zirconia. Specific examples include erbium ion and neodymium ion. These are stabilizers and are colorants.

At least one of the metal ion contained in the impregnating solution may be a transition metal ion. Specific transition metal ion is an iron ion. By containing a transition metal ion, it is possible to color a zirconia sinterd body. The metal ion may be only a transition metal ion.

At least one of the metal ion contained in the impregnating solution may be a rare earth metal ion. Specific examples of the rare earth metal ion include an yttrium ion. By containing a rare earth metal ion, it is possible to improve the translucency of the zirconia sintered body and to color the zirconia sintered body. The metal ion may be only a rare earth metal ion.

At least one of the metal ion contained in the impregnating solution may be any one or more of aluminum ion, gallium ion and indium ion. By containing any one or more of aluminum ion, gallium ion and indium ion, it is possible to improve the sinterability of the zirconia sintered body and to improve the translucency of the zirconia sintered body. The metal ion may be only aluminum ion, gallium ion and/or indium ion. The metal ion may be only a rare earth metal ion, a transition metal ion, aluminum ion, gallium ion and/or indium ion.

The metal ion source of the present invention is preferably a metal ion organic acid salt from the viewpoint of low decomposition temperature and low contamination of the firing furnace. Examples include acetate and citrate. Organic acid salts decompose at lower temperatures than inorganic salts such as halogen compounds, nitrates, and sulfates. In the case that the decomposition temperature is high, there is a case where it is difficult to impart sufficient translucency and strength to a zirconia perfect sintered body because pore remains in the sintering process.

There is no particular limitation on the concentration of a metal ion in the impregnating solution in the present invention. It is preferable that the metal ion concentration is determined by the desired metal supporting amount and the solubility of the metal ion source in the solvent.

The solvent used in the impregnating solution in the present invention is not particularly limited, but water is preferable since it is easily available and is easy to handle.

The impregnating solution of the present invention may be added with acid, base, or pH adjusting agent for the purpose of controlling the pH. The acid, base or pH adjusting agent to be used is not particularly limited, but it is possible to use an organic acid such as acetic acid, citric acid and the like or aqueous ammonia from the viewpoint of leaving no residue on the zirconia mill blank for dental cutting and machining.

The zirconium component in the impregnating solution can be any one or more of zirconium oxychloride, zirconium oxyacetate, and zirconyl nitrate. Such zirconium components are easily available and easy to handle.

The impregnating solution may contain a precipitant. A precipitant precipitates a metal compound by heat treatment or other operation after permeation of the impregnating solution, and specific examples include urea and hexamethylenetetetramine. Urea may be used since it is easily available and is easy to handle. By this, segregation of a metal compound can be suppressed.

An amount of the precipitant in the impregnating solution of the present invention is preferably an amount capable of depositing the entire amount of the metal ion in the impregnating solution. When the amount of the precipitant is small, there is a tendency that the time required for depositing the entire amount of the metal ion is long, and when the amount of the precipitant is large, there is a tendency that the time required for depositing the entire amount of the metal ion is short. However, in both cases, there is no influence to the effect of the present invention. When the amount of the precipitant is less than the amount capable of depositing the entire amount of the metal ion in the impregnating solution, there is a case where the entire amount of the metal ion is not deposited and the metal ion segregates in the subsequent drying step, and therefore it is not preferable. When the amount of the precipitant is excessive, there is no influence to the effect of the present invention, however, a large amount of the precipitant remains after the depositing step, and therefore it is not preferable.

The impregnating solution may contain various additives. Examples of additives include glycols and polymers that adjust the viscosity of the impregnating solution, and chelating agents that increase the stability of a metal ion. Specific Examples of the former include ethylene glycol, propylene glycol, polyethylene glycol and the like. Specific Examples of the latter include citric acid, ammonium ethylenediaminetetraacetate and the like.

The zirconium component in the impregnating solution may include a hydroxy acid. The hydroxy acid may be, for example, a citric acid, a malic acid and a lactic acid. By containing such hydroxy acid, it is possible to suppress segregation of a metal compound after drying.

As a method of permeatiing the zirconia calcined body with the impregnating solution, any method can be used without particular limitation as long as it is possible to control a position and an amount of the impregnating solution in the the zirconia calcined body. In a simple and preferable method, after permiating the predeterminend amount of the first type impregnating solution from one end, the predeterminend amount of the second type impregnating solution is impregnated from the same end or the other end.

The atmosphere in impregnating the impregnating solution is not particularly limited, and an ambient air, an inert gas and the like can be used under normal pressure, reduced pressure, and pressurized conditions. Since no special facilities is required, it is preferable to perform under normal pressure in an ambient air.

The metal ion concentration of the impregnating solution is not particularly limited, but in the case of an yttrium ion, for example, it may be about within a range of 2.0 mass% to 10.0 mass%. When it is 2.0 mass% or less, there is a case that the amount of supported yttrium is small and sufficient characteristic is not obtained. When it is 10.0 mass% or more, there is a case that the amount of supported yttrium is large and physical property is adversely affected. Since the optimum amount of supported metal changes depending on the type of metal and the desired characteristics, the metal ion concentration of the impregnating solution is preferably determined from the desired amount of supported metal and the solubility of a metal ion source in the solvent.

As the method of permiating the zirconia calcined body with the impregnating solution, any method can be used without particular limitation as long as the impregnating solution can infiltrate into the pores of the zirconia mill blank for dental cutting and machining. In a simple and preferable method, the whole and/ or a part of the zirconia mill blank for dental cutting and machining is immersed in the impregnating solution. In this case, the impregnating solution can permiate by the capillarity into an inside of the zirconia mill blank for dental cutting and machining. The immersion time is 3 minutes or more, preferably 10 minutes to 10 hours.

In a specific example of using a plurality of impregnating solutions, after permiating the first type impregnating solution from one end, the second type impregnating solution is impregnated from the same end or the other end.

It is possible to comprise a drying step after the permiating step. The drying step is a step of drying the porous zirconia molded body in which a metal compound is deposited. The drying temperature may be within a range of 50 to 200 °C, and the drying time may be within a range of 15 minutes to 20 hours. It is possible to sufficiently vaporize the solvent after drying. In some cases, the heat treatment step may be carried out following this drying step. The drying step may also be included in the heat treatment step.

It is possible to comprise a degreasing step after the permiating step. The degreasing step is a step of removing unnecessary components such as an organic matter contained in the impregnating solution. The degreasing temperature may be within a range of 200 to 600 °C, and the degreasing time may be within a range of 50 to 200 hours. It is possible to completely remove unnecessary components by degreasing. In some cases, the heat treatment step may be carried out following this degreasing step. The degreasing step may also be included in the heat treatment step.

In a case in which the impregnating solution contains a precipitant, it is preferable, after impregnation, to comprise a depositing step in which the precipitant is deposited to precipitate a compound containing a metal ion.

There is no particular limitation on the method of decomposing a precipitant. Examples include hydrolysis by heating and hydrolysis by enzymes, and hydrolysis by heating is preferable because of its simplicity.

The method of hydrolysis by heating is not particularly limited as long as it can maintain a constant temperature. Examples include a method of using a thermostat, a dryer, a water bath and an oil bath.

It is preferable to seal the zirconia calcined body in order to prevent a solvent from evaporating during hydrolysis by heating. The method is not particularly limited, and examples thereof include a method of covering with a resin sheet and degassing in some cases.

The temperature of hydrolysis by heating is not particularly limited as long as it is equal to or higher than the decomposition temperature of a precipitant. However, when it is too low, a long heat treatment is required until the entire amount of a metal compound is deposited, and therefore it is not preferable. Further, when the temperature exceeds the boiling point of a solvent, the solvent evaporates during the heat treatment and a supported metal compound segregates, and therefore it is not preferable.

The time required for hydrolysis by heating changes depending on a metal ion concentration, a precipitant concentration, a heat treatment temperature and the like, and therefore it is necessary to be adjusted appropriately, but it is generally about 10 minutes to several days. It is preferably 10 minutes to 24 hours.

Since a solvent, unreacted product, by-product and the like remain in the zirconia calcined body in which a metal compound is deposited and supported, prepared in such a way, it is possible to perform a heat treatment. The heat treatment method is not particularly limited, but heat treatment under normal atmospheric pressure may be used since no special facilities is required. The heat treatment temperature is not particularly limited, but may be within a range of 500 °C to 1200 °C. By heat treatment, impurities, organic substances and odors can be removed.

By the preparing method of a zirconia mill blank for dental cutting and machining of the present invention, it is possible to prepare, for example, a zirconia mill blank for dental cutting and machining of the present invention. Specifically, the zirconia mill blank for dental cutting and machining of the present invention is a zirconia mill blank for dental cutting and machining wherein in a case in which, in bewteen two opposing surfaces, a section from one end to 50% of a distance between the two opposing surfaces is defined as section A, a section from other end to 50% of the distance between the two opposing surfaces is defined as section B, the section A contains a region A in which a compound of a stabilizer element is supported in a pore, the section B contains a region B in which a zirconium compound is supported in a pore, and a concentration of a metal ion in the region A and a concentration of a metal ion in the region B are different and/or a type of a metal ion in the region A and a type of a metal ion in the region B are different.

A stabilizer concentration in the zirconium compound may be lower than a stabilizer concentration in the zirconia mill blank for dental cutting and machining which constitutes a base material.

There is no restriction on a stabilizer element, but it may be a rare earth metal because it is often used in the zirconia mill blank for dental cutting and machining, and among them, it may be yttrium. As other stabilizer elements, erbium oxide, neodymium oxide, praseodymium oxide and terbium oxide which also have the effect of a coloring material may be used.

It is preferable that a compound of a coloring element is supported in the pore of at least one of the region A and the region B. There is no particular designation for the coloring element, but it may be a rare earth metal and/or a transition metal, and iron, nickel, cobalt, manganese, erbium, praseodymium, terbium, neodymium, vanadium and the like are preferable because they are often used in the dental zirconia.

At least one of an aluminum compound, a gallium compound and an indium compound may be supported in the pore of at least one of the region A and the region B. By supporting these compounds, it is possible to change sintering behavior of the zirconia mill blank for dental cutting and machining.

At least one of a niobium compound and a tantalum compound may be supported in the pore of at least one of the region A and the region B. When these compounds are supported, it is possible to improve fracture toughness of the zirconia perfect sintered body.

The difference in relative density between the region A and the region B is preferably less than 0.008, more preferably less than 0.005, and particular preferably less than 0.003. When the difference in relative density exceeds 0.008, there is a tendency that fitting property of the prepared dental prosthesis deteriorates.

As the zirconia powder used for preparing the zirconia mill blank for dental cutting and machining of the present invention, any known zirconia powder may be used without any limitations. Specifically, it is preferable that zirconia powder used for preparing the zirconia mill blank for dental cutting and machining of the present disclosure is prepared by a hydrolysis method. More specifically, the zirconia powder is prepared by a method which comprises heating a solution in which a zirconium salt and a yttrium compound are mixed and dissolved to cause a hydrolysis reaction, drying and firing the generated sol to prepare a zirconia powder, and pulverizing and granulating the powder. Further, by mixing an aluminum compound before this pulverization step, an alumina-containing zirconia powder is prepared.

The primary particle diameter of a zirconia powder used for preparing the zirconia mill blank for dental cutting and machining in the present invention may be within a range of 1 to 500 nm. When the primary particle diameter is less than 1 nm, there is a tendency that it is difficult to impart sufficient strength, although translucency of the zirconia sintered body is improved. On the other hand, when the primary particle diameter is more than 500 nm, there is a tendency that it is difficult to impart sufficient strength to the zirconia sintered body.

The zirconia mill blank for dental cutting and machining of the present invention may contain a coloring material. Specific examples thereof include iron oxide for imparting yellow color and erbium for imparting red color. In addition, there is no problem at all even if a coloring material containing an element such as cobalt, manganese and chromium for adjusting color in addition to these coloring materials is used together. In the present invention, the tooth color can be easily imparted by including the coloring material.

The relative density of the zirconia sintered body prepared by firing the zirconia mill blank for dental cutting and machining of the present invention at 1550 °C may be 98% or more of the theoretical density. The relative density is determined by the measured density/the theoretical density. When the relative density is less than 98%, there is a tendency that strength and translucency are lowered.

A crystal phase of the zirconia mill blank for dental cutting and machining of the present invention may be tetragonal and/ or cubic. When the crystal phase is monoclinic phase, there is a case in which sufficient translucency is not imparted after perfect sintering of zirconia.

A preparing method of the zirconia mill blank for dental cutting and machining of the present invention is not particularly limited, and any known preparing methods can be used without any problem. Specifically, it is preferable to be prepared by molding a zirconia powder by press molding. Furthermore, it may be prepared by a multilayer molding in which zirconia powders having different color tones or compositions are press-molded in multiple stages.

The zirconia mill blank for dental cutting and machining of the present invention may be subjected to isostatic pressing by cold isostatic pressing (CIP treatment) after the press molding.

The maximum load pressure of CIP treatment in the present invention may be 50 Mpa or more. When the maximum load pressure is less than 50 MPa, there is a case where sufficient translucency and strength is not imparted to the zirconia sintered body.

The holding time at the maximum load pressure of the CIP treatment in the present invention is not particularly limited, but in general, may be within a range of 0 to 150 seconds and may be within a range of 0 to 60 seconds.

The time period required for the series of processes from the start of pressurization to the end of depressurization in the CIP treatment is not particularly limited, but may be usually within a range of 30 seconds to 10 minutes and may be within a range of 3 minutes to 7 minutes. When the time is too short, a molding body may be destroyed, and when the time is too long, production efficiency worsens, and therefore these are not preferable.

A calcination temperature of the zirconia mill blank for dental cutting and machining of the present invention is preferably within a range of 800 to 1200 °C. When the calcination temperature is less than 800 °C, because Vickers hardness and/ or bending strength become too low and therefore there is a tendency that chipping and breakage easily occur in the cutting and machining. On the other hand, when the calcination temperature is more than 1200 °C, because Vickers hardness and/ or bending strength become too high and therefore there is a tendency that a milling bar of a milling machine is heavily consumed to raise a running cost.

By such preparing method, a zirconia mill blank for dental cutting and machining of the present invention can be prepared. The prepared zirconia mill blank for dental cutting and machining is severed, cut, and surface polished so as to have a desired size as necessary.

The method for perfect sintering the zirconia mill blank for dental cutting and machining of the present invention is not particularly limited, but a simple and preferred method is to firing at normal pressure. The firing temperature is not limited, but may be within a range from 1450 to 1600 °C, and may be within a range from 1500 to 1600 °C. The holding time at the firing temperature is not particularly limited, but may be within a range of 1 minute to 12 hours, and may be within a range of 2 to 4 hours. The temperature increase rate is not particularly limited, but may be within a range from 1 to 400°C/min and from 3 to 100°C/h.

The type of a prosthesis device prepared by cutting and machining the zirconia mill blank for dental cutting and machining according to the present invention is not limited particularly, and there is no problem at all even if the prosthesis device is any of an inlay, an onlay, a veneer, a crown, a bridge and the like. Therefore, a shape of a zirconia mill blank for dental cutting and machining which is cut and machined for preparing a prosthesis device is not limited particularly, and any zirconia mill blank for dental cutting and machining can be used even if the zirconia mill blank for dental cutting and machining has any shape such as a block shape corresponding to an inlay, an onlay, a veneer, a crown and the like and a disk shape corresponding to a bridge.

[Examples]

Hereinafter, the present invention is described by way of Examples in more detail, and specifically, but the present invention is not limited to these Examples.

(Preparation of zirconia calcined body)

Zirconia powder containing 5.5 mol% of solid-solved yttrium in an amount of 486g was filled in a mold (φ100 mm), and press molding (surface pressure: 50 MPa) was performed to obtain a molded body. Further, the molded body was subjected to CIP treatment (maximum load pressure: 200 MPa, holding period: 1 minute). Thereafter, calcination was performed in an electric furnace (1000 °C, 30 minutes) to prepare a zirconia calcined body having a diameter of 98.5 mm and a thickness of 18 mm.

(Preparation of impregnating solution)

Table 1 and Table 2 show the compositions of the impregnating solutions. According to the compositions shown in Table 1 and Table 2, solutions in amount of 100 g were prepared by adding a metal salt and an additive to ion-exchanged water and stirred for 1 hour, and were used as impregnating solutions.

[Table 1]

|  | Solution 1 | Solution 2 | Solution 3 | Solution 4 |
|---|---|---|---|---|
| Solvent | Ion exchange water | Ion exchange water | Ion exchange water | Ion exchange water |
| Metal salt 1 | Yttrium nitrate n-hydrate | Iron(III) nitrate nonahydrate | Iron(III) nitrate nonahydrate | Iron(III) nitrate nonahydrate |
| Metal ion concentration 1 (wt%) | 7.7 | 0.17 | 0.23 | 0.17 |
| Metal salt 2 | Praseodymium nitrate n-hydrate | Nickel nitrate hexahydrate | Nickel nitrate hexahydrate | Nickel nitrate hexahydrate |
| Metal ion cocentration 2 (wt%) | 0.3 | 0.09 | 0.13 | 0.09 |
| Metal salt 3 | Neodymium nitrate hexahydrate | Zirconium oxyacetate | Zirconium oxyacetate | Zirconium oxyacetate |
| Metal ion cocentration 3 (wt%) | 0.18 | 7 | 7 | 6 |
| Additive 1 | Propylene glycol 25mass% | Propylene glycol 25mass% | Pronylene glycol 25mass% | Propylene glycol 25mass% |
| Additive 2 |  | - | - | - |
| Ctotal | 8.18 | 7.26 | 7.36 | 6.26 |

|  | Solution 5 | Sontion 6 | Solution 7 | Solution 8 |
|---|---|---|---|---|
| Solvent | Ion exchange water | Ion exchange water | Ion exchange water | Ion exchange water |
| Metal salt 1 | Iron(III) nitrate nonahydrate | Iron(III) nitrate nonahydrate | Iron(III) nitrate nonahydrate | Iron(III) nitrate nonahydrate |
| Metal ion cocentration 1 (wt%) | 0.23 | 0.17 | 0.23 | 0.17 |
| Metal salt 2 | Nickel nitrate hexahydrate | Nickel nitrate hexahydrate | Nickel nitrate hexahydrate | Nickel nitrate hexahydrate |
| Metal ion cocentration 2 (wt%) | 0.13 | 0.09 | 0.13 | 0.09 |
| Metal salt 3 | Zirconium oxyacetate | Zirconium oxyacetate | Zirconium oxyacetate | Zirconium oxyacetate |
| Metal ion cocentration 3 (wt%) | 6 | 5.7 | 5.7 | 4 |
| Additive 1 | Propylene glycol 25mass% | Propylene glycol 25mass% | Pronylene glycol 25mass% | Propylene glycol 25mass% |
| Additive 2 |  | - | - | - |
| Ctotal | 6.36 | 5.96 | 6.06 | 4.26 |

|  | Solution 9 | Solution 10 | Solution 11 | Solution 12 |
|---|---|---|---|---|
| Solvent | Ion exchange water | Ion exchange water | Ion exchange water | Ion exchange water |
| Metal salt 1 | Iron(III) nitrate nonahydrate | Iron(III) nitrate nonahydrate | Iron(III) nitrate nonahydrate | Iron(III) nitrate nonahydrate |

(continued)

|  | Solution 9 | Solution 10 | Solution 11 | Solution 12 |
|---|---|---|---|---|
| Solvent | Ion exchange water | Ion exchange water | Ion exchange water | Ion exchange water |
| Metal ion cocentration 1 (wt%) | 0.23 | 0.17 | 0.23 | 0.17 |
| Metal salt 2 | Nickel nitrate hexahydrate | Nickel nitrate hexahydrate | Nickel nitrate hexahydrate | Nickel nitrate hexahydrate |
| Metal ion cocentration 2 (wt%) | 0.13 | 0.09 | 0.13 | 0.09 |
| Metal salt 3 | Zirconium oxyacetate | Zirconium oxyacetate | Zirconium oxyacetate | Zirconium oxyacetate |
| Metal ion cocentration 3 (wt%) | 4 | 3.7 | 3.7 | 3 |
| Additive 1 | Propylene glycol 25mass% | Propylene glycol 25mass% | Pronylene glycol 25mass% | Propylene glycol 25mass% |
| Additive 2 | - | - |  | - |
| Ctotal | 4.36 | 3.96 | 4.06 | 3.26 |
|  |  |  |  |  |

|  | Solution 13 | Solution 14 | Solution 15 | Solution 16 |
|---|---|---|---|---|
| Solvent | Ion exchange water | Ion exchange water | Ion exchange water | Ion exchange water |
| Metal salt 1 | Iron(III) nitrate nonahydrate | Iron(III) nitrate nonahydrate | Iron(III) nitrate nonahydrate | Iron(III) nitrate nonahydrate |
| Metal ion cocentration 1 (wt%) | 0.23 | 0.17 | 0.23 | 0.17 |
| Metal salt 2 | Nickel nitrate hexahydrate | Nickel nitrate hexahydrate | Nickel nitrate hexahydrate | Nickel nitrate hexahydrate |
| Metal ion cocentration 2 (wt%) | 0.13 | 0.09 | 0.13 | 0.09 |
| Metal salt 3 | Zirconium oxyacetate | - |  | Zirconium oxyacetate |
| Metal ion cocentration 3 (wt%) | 3 | - |  | 2.7 |
| Additive 1 | Propylene glycol 25mass% | Propylene glycol 25mass% | Pronylene glycol 25mass% | Propylene glycol 25mass% |
| Additive 2 |  | - |  |  |
| Ctotal | 3.36 | 0.26 | 0.36 | 2.96 |

[Table 2]

|  | Solution 17 | Solution 18 | Solution 19 | Solution 20 |
|---|---|---|---|---|
| Solvent | Ion exchange water | Ion exchange water | Ion exchange water | Ion exchange water |
| Metal salt 1 | Iron(III) nitrate nonahydrate | Yttrium nitrate n-hydrate | Iron(III) nitrate nonahydrate | Iron(III) nitrate nonahydrate |

(continued)

| | Solution 17 | Solution 18 | Solution 19 | Solution 20 |
|---|---|---|---|---|
| Solvent | Ion exchange water | Ion exchange water | Ion exchange water | Ion exchange water |
| Metal ion concentration 1 (wt%) | 0.23 | 7.7 | 0.17 | 0.23 |
| Metal salt 2 | Nickel nitrate hexahydrate | Praseodymium nitrate n-hydrate | Nickel nitrate hexahydrate | Nickel nitrate hexahydrate |
| Metal ion concentration 2 (wt%) | 0.13 | 0.3 | 0.09 | 0.13 |
| Metal salt 3 | Zirconium oxyacetate | Neodymium nitrate hexahydrate | Zirconium oxyacetate | Zirconium oxyacetate |
| Metal ion concentration 3 (wt%) | 2.7 | 0.18 | 7 | 7 |
| Additive 1 | Propylene glycol 25mass% | Urea 34mass% | Urea 37mass% | Urea 37mass% |
| Additive 2 | - | - | - | - |
| Ctotal | 3.06 | 8.18 | 7.26 | 736 |

| | Solution 21 | Solution 22 | Soiation 23 | Solution 24 |
|---|---|---|---|---|
| Solvent | Ion exchange water | Ion exchange water | Ion exchange water | Ion exchange water |
| Metal salt 1 | Indium nitrate trihydrate | Iron(III) nitrate nonahydrate | Iron(III) nitrate nonahydrate | Yttrium nitrate n-hydrate |
| Metal ion concentration 1 (wt%) | 8.5 | 0.17 | 0.23 | 7 |
| Metal salt 2 | Praseodymium nitrate n-hydrate | Nickel nitrate hexahydrate | Nickel nitrate hexahydrate | Praseodymium nitrate n-hydrate |
| Metal ion concentration 2 (wt%) | 0.3 | 0.09 | 0.13 | 0.3 |
| Metal salt 3 | Neodymium nitrate hexahydrate | Zirconium oxyacetate | Zirconium oxyacetate | Gallium nitrate n-hydrate |
| Metal ion concentration 3 (wt%) | 0.18 | 8 | 8 | 1.5 |
| Additive 1 | Propylene glycol 25mass% | Propylene glycol 25mass% | Pronylene glycol 25mass% | Propylene glycol 25mass% |
| Additive 2 | - | - | - | - |
| Ctotal | 8.98 | 8.26 | 8.36 | 8.8 |

| | Solution 25 | Solution26 | Solution 27 | Solution 28 |
|---|---|---|---|---|
| Solvent | Ion exchange water | Ion exchange water | Ion exchange water | Ion exchange water |
| Metal salt 1 | Iron(III) nitrate nonahydrate | Iron(III) nitrate nonahydrate | Iron(III) nitrate nonahydrate | Iron(III) nitrate nonahydrate |

(continued)

|  | Solution 25 | Solution26 | Solution 27 | Solution 28 |
|---|---|---|---|---|
| Solvent | Ion exchange water | Ion exchange water | Ion exchange water | Ion exchange water |
| Metal ion concentration 1 (wt%) | 0.17 | 0.23 | 0.17 | 0.23 |
| Metal salt 2 | Gallium nitrate n-hydrate | Gallium nitrate n-hydrate | Aluminum nitrate nonahydrate | Aluminum nitrate nonahydrate |
| Metal ion concentration 2 (wt%) | 1.5 | 1.5 | 0.3 | 0.3 |
| Metal salt 3 | Zirconium oxyacetate | Zirconium oxyacetate | Zirconium oxyacetate | Zirconium oxyacetate |
| Metal ion concentration 3 (wt%) | 7 | 7 | 7 | 7 |
| Additive 1 | Propylene glycol 25mass% | Propylene glycol 25mass% | Pronylene glycol 25mass% | Propylene glycol 25mass% |
| Additive 2 | - | - | - | - |
| Ctotal | 8.67 | 8.73 | 7.47 | 7.53 |

|  | Solution 29 | Solution30 | Solution 31 | Solution 32 |
|---|---|---|---|---|
| Solvent | Ion exchange water | Ion exchange water | Ion exchange water | Ion exchange water |
| Metal salt 1 | Yttrium nitrate n-hydrate | Yttrium nitrate n-hydrate | Iron(III) nitrate nonahydrate | Iron(III) nitrate nonahydrate |
| Metal ion concentration 1 (wt%) | 4 | 7.7 | 0.17 | 0.23 |
| Metal salt 2 | Praseodymium nitrate n-hydrate | Praseodymium nitrate n-hydrate | Nickel nitrate hexahydrate | Nickel nitrate hexahydrate |
| Metal ion concentration 2 (wt%) | 0.3 | 0.3 | 0.09 | 0.13 |
| Metal salt 3 | Neodymium nitrate hexahydrate | Neodymium nitrate hexahydrate | Zirconium oxychloride octahydrate | Zirconium oxychloride octahydrate |
| Metal ion concentration 3 (wt%) | 0.18 | 0.18 | 7 | 7 |
| Additive 1 | Propylene glycol 25mass% | Citric acid monohydrate 25mass% | Citric acid monohydrate 25mass% | Citric acid monohydrate 25mass% |
| Additive 2 |  |  |  |  |
| Ctotal | 4.48 | 8.18 | 7.26 | 736 |

(Impregnation of impregnating solution into zirconia calcined body)

The zirconia calcined body was placed on a horizontal workbench, and a jig was attached so that the impregnating solution could be held on the top surface of the zirconia calcined body. The impregnating solution (1) (solution (1)) was poured into the top surface of the zirconia calcined body in the specified amount described in Table 3 and allowed to stand until the entire amount was absorbed by the zirconia calcined body. The impregnating solution (II) (solution (II)) was poured into the top surface of the zirconia calcined body in the specified amount described in Table 3 and allowed to stand until the entire amount was absorbed by the zirconia calcined body. The impregnating solution (III) (solution (III)) was poured into the top surface of the zirconia calcined body in the specified amount described in Table 3 and allowed to stand until the entire amount was absorbed by the zirconia calcined body. The above operation was performed for all impregnating solutions.

(Heat treatment after impregnation)

In the case that the impregnating solution (solution) contained urea, heat treatment was performed after impregnation of the impregnating solution. The zirconia calcined body after impregnation was placed in a resin bag and degassed. The zirconia calcined body placed in the resin bag and degassed was placed in a dryer, and then a heat treatment at 95 °C for 15 hours was performed to deposit a metal compound. After the heat treatment, the zirconia calcined body was taken out from the resin bag and dried (120 °C, 1 hour) to prepare a zirconia calcined body supporting a metal compound.

(Removal of residual organic matter)

The zirconia calcined body after impregnation and/or heat treatment was heat treated in an electric furnace (500 °C, 30 min) to prepare a zirconia mill blank for dental cutting and machining in which a metal compound is supported in a pore.

(Measurement of relative density)

By using a dental milling machine (DWX51D, manufactured by Roland Corporation), the surface to which the impregnating solution was poured during impregnation was defined as the surface B, the surface of opposite side was defined as the surface A, the surface which was parallel to the surface A and was positioned at 1.0 mm from the surface A toward the surface B was defined as the surface C, and the surface which was parallel to the surface B and was positioned at 1.0 mm from the surface B toward the surface A was defined as the surface D. A test specimen having 5 mm x 5 mm x 5 mm was prepared from the surface C toward the surface B and used as a test specimen for the region A. In the same manner, a test specimen having 5 mm x 5 mm x 5 mm was prepared from the surface D toward the surface A and used as a test specimen for the region B. The relative density of each test specimen was calculated using the following formula.

$$\text{Relative density} = (\rho/\rho0)$$

$$\rho0 = 100/[\{(A1/\rho A1) + (A2/\rho A2) + ... + (An/\rho An)\} + \{100\text{-}(A1 + A2 + ... + An)\}/\rho]$$

In the formula, the "$\rho$" is the measured density of the test specimen, the "$\rho0$" is the theoretical density of the test specimen, the "An" is the content of substances other than zirconia and yttria (wt%), the "$\rho$An" is the theoretical density of the substances other than zirconia and yttria, and the "$\rho$" is the theoretical density of yttria stabilized zirconia.
The above "$\rho$" changes depending on the content of yttria and the crystalline phase of zirconia, and in the present specification, the "$\rho$" is determined by the formula described in "Lattice and Density for Y2O3-Stabilized ZrO2." J. Am. Ceram. Soc. 1986, 69(4 ), 325-332.

(Evaluation of supported elements)

By using a dental milling machine (DWX51D, manufactured by Roland Corporation), the surface to which the impregnating solution was poured during impregnation was defined as the surface B, the surface of opposite side was defined as the surface A, the surface which was parallel to the surface A and was positioned at 1.0 mm from the surface A toward the surface B was defined as the surface C, and the surface which was parallel to the surface B and was positioned at 1.0 mm from the surface B toward the surface A was defined as the surface D. A disk-shap test specimen having φ 25 mm x 2.0 mm was prepared from the surface C toward the surface B and used as a test specimen for the region A. In the same manner, a disk-shap test specimen having φ 25 mm x 2.0 mm was prepared from the surface D toward the surface A and used as a test specimen for the region B. Compositon analysis of each test specimen was performed by using a fluorescent X-ray analysis device (manufactured by Rigaku Corporation). Measurements were performed on the top surface and the bottom surface of each test specimen, and the average of these measurements was used as the content ratio of each element in each test specimen. The content ratio (mass%) for all elements are shown in terms of oxide. Similar measurements were performed for the calcined body without impregnating liquid (unimpregnated calcined body). By using the results, the element that satisfies the following formula was defined as a supported element.

(content ratio of each element in each test specimen (mass%) x the weight of each test specimen (g) -

content ratio of each element in the test specimen in the calcined body without impregnating liquid

(mass%) x the weight of the test specimen in the calcined body without impregnating liquid (g)) /

(content ratio of each element in each test specimen (mass%) x the weight of each test specimen (g))

> 0.02

There is no particular restriction on the method for measuring the content (mass%) of substances other than zirconia and yttria contained in the test specimen. In the present specification, the content was measured by a fluorescent X-ray analysis device (manufactured by Rigaku Corporation).

(Measurement of shrinkage)

By using a dental milling machine (DWX51D, manufactured by Roland Corporation), a test specimen having 15 mm (x: direction parallel to the surface C) x 15 mm (y: direction parallel to the surface C) x 2.0 mm (z: direction perpendicular to the surface C) was prepared from the surface C toward the surface B and used as a test specimen for an enamel portion. In the same manner, the surface which was parallel to the surface A and waspositioned at 8.0 mm from the surface A was defined as the surface E, a test specimen having 15 mm (x: direction parallel to the surface E) x 15 mm (y: direction parallel to the surface E) x 2.0 mm (z: direction perpendicular to the surface E) was prepared from the surface E toward the surface B and used as a test specimen for a central portion. In the same manner, a test specimen having 15 mm (x: direction parallel to the surface D) x 15 mm (y: direction parallel to the surface D) x 2.0 mm (z: direction perpendicular to the surface D) was prepared from the surface D toward the surface A and used as a test specimen for a servical portion. The dimensions of each specimen before and after perfect sintering were measured using a micrometer, and the shrinkage rate of each area was calculated using the following formula. AUSTROMAT 674i (manufactured by DEKEMA) was used for sintering the test specimens (sintering temperature: 1550°C, holding time: 1 hour).

$$\text{Shrinkage rate } x = x \text{ (mm) before perfect sintering} / x \text{ (mm) after perfect sintering}$$

$$\text{Shrinkage rate } y = y \text{ (mm) before perfect sintering} / y \text{ (mm) after perfect sintering}$$

$$\text{Shrinkage rate } z = z \text{ (mm) before perfect sintering} / z \text{ (mm) after perfect sintering}$$

$$\text{Shrinkage ratio } z = z \text{ (mm) before complete sintering} / z \text{ (mm) after complete sintering}$$

$$\text{Average of shrinkage rate} = (\text{Shrinkage rate } x + \text{Shrinkage rate } y + \text{Shrinkage rate } z) / 3$$

(Evaluation of fitting property)

Six-tooth bridge from maxillary left canine tooth to maxillary right canine tooth was machined from the prepared zirconia mill blank for dental cutting and machining by using a dental milling machine (DWX51D, manufactured by Roland Corporation), and was perfect sintered (sintering temperature: 1550°C, holding time: 1 hour) by using AUSTROMAT 674i (manufactured by DEKEMA) to prepare a zirconia six-tooth bridge. The prepared zirconia six-tooth bridge was mounted on a plaster model, and was checked for gap in margin area, and the presence or absence of rattle, and evaluated for fitting property according to the following evaluation criteria. The observations and evaluations were performed by five technicians, and the results of the most frequent evaluations are shown in Table 3.

AA: There are no gaps or rattles in the margin, and it can be used with no or almost no additional adjustment.
A: There are almost no gaps or rattles in the margin, and it can be used with slight modifications.
B: There are gaps and rattles in the margin, but it can be used with some modification.
C: There are large gaps and rattles in the margin, and it is unusable or requires major modification.

(Evaluation of aesthetic property)

Six-tooth bridge from maxillary left canine tooth to maxillary right canine tooth was machined from the prepared zirconia mill blank for dental cutting and machining by using a dental milling machine (DWX51D, manufactured by Roland Corporation), and was perfect sintered (sintering temperature: 1550°C, holding time: 1 hour) by using AUSTROMAT 674i (manufactured by DEKEMA) to prepare a zirconia six-tooth bridge. The appearance of the six-tooth bridge bridges was observed and aesthetic property was evaluated according to the following evaluation criteria. The observations and evaluations were performed by five technicians, and the results of the most frequent evaluations are shown in Table 3.

∘: It has translucency, color tone and their gradations similar to a natural tooth, and exhibits particularly excellent aesthetic property.
x: Some or all of transparency, color tone and their gradations deviate from natural tooth and it didnot show excellent aesthetic property.

Table 3 shows the evaluation results of the prepared zirconia mill blank for dental cutting and machining in Examples and Comparative Examples.

[Table 3]

| | | Example 1 | Example 2 | Example 3 | Example 4 | Example 5 | Example 6 | Example 7 | Example 8 |
|---|---|---|---|---|---|---|---|---|---|
| Stabilizer concentration in base material (mol%) | | 5.5 | 5.5 | 5.5 | 5.5 | 5.5 | 5.5 | 5.5 | 5.5 |
| Type of impregnating liquid | Solution (I) | Solution 1 | Solution 1 | Solution 1 | Solution 1 | Solution 1 | Solution 1 | Solution 18 | Solution 1 |
| | Solution (II) | Solution 2 | Solution 4 | Solution 6 | Solution 8 | Solution 10 | Solution 12 | Solution 19 | Solution 2 |
| | Solution (III) | Solution 3 | Solution 5 | Solution 7 | Solution 9 | Solution 11 | Solution 13 | Solution 20 | Solution 3 |
| Amount of impregnating liquid (g) | Solution (I) | 23.0 | 23.0 | 23.0 | 23.0 | 23.0 | 23.0 | 23.0 | 23.0 |
| | Solution (II) | 23.0 | 23.0 | 23.0 | 23.0 | 23.0 | 23.0 | 23.0 | 23.0 |
| | Solution (III) | 23.0 | 23.0 | 23.0 | 23.0 | 23.0 | 23.0 | 23.0 | 23.0 |
| Ctotal of impregnation liquid | Solution (I) | 8.180 | 8.180 | 8.180 | 8.180 | 8.180 | 8.180 | 8.180 | 8.180 |
| | Solution (II) | 7.260 | 6.26 | 5.96 | 4.26 | 3.96 | 3.26 | 7.260 | 7.260 |
| | Solution (III) | 7360 | 6.36 | 6.06 | 4.36 | 4.06 | 3.36 | 7.360 | 7.360 |
| △Ctotal | 含浸液 (I)-(II) | 0.920 | 1.920 | 2.220 | 3.920 | 4.220 | 4.920 | 0.920 | 0.920 |
| | 含浸液 (II)-(III) | 0.100 | 0.100 | 0.100 | 0.100 | 0.100 | 0.100 | 0.100 | 0.100 |
| | 含浸液 (I)-(III) | 0.820 | 1.820 | 2.120 | 3.820 | 4.120 | 4.820 | 0.820 | 0.820 |
| Supported element | Section A | Y, Pr, Nd | Y, Pr, Nd | Y, Pr, Nd | Y, Pr, Nd | Y, Pr, Nd | Y, Pr, Nd | Y, Pr, Nd | Y, Pr, Nd |
| | SectionB | Zr, Fe, Ni | Zr, Fe, Ni | Zr, Fe, Ni | Zr, Fe, Ni | Zr, Fe, Ni | Zr, Fe, Ni | Zr, Fe, Ni | Zr, Fe, Ni |
| Stabilizer concentration in zirconium compounds | | 0.0 | 0.0 | 0.0 | 0.0 | 0.0 | 0.0 | 0.0 | 0.0 |
| Relative density | SectionA | 0.567 | 0.567 | 0.567 | 0.567 | 0.567 | 0.567 | 0.566 | 0.567 |
| | Section B | 0.566 | 0.565 | 0.564 | 0.563 | 0.562 | 0.560 | 0.567 | 0.565 |
| | Difference | 0.001 | 0.002 | 0.003 | 0.004 | 0.005 | 0.007 | 0.001 | 0.002 |
| Shrinkage rate | Enamel | 1.208 | 1.208 | 1.208 | 1.208 | 1.208 | 1.208 | 1.209 | 1.208 |
| | Central | 1.209 | 1.210 | 1.211 | 1.211 | 1.212 | 1.213 | 1.209 | 1.209 |
| | Servical | 1.209 | 1.210 | 1.210 | 1.211 | 1.212 | 1.213 | 1.208 | 1.210 |
| | Average | 1.209 | 1.209 | 1.210 | 1.210 | 1.211 | 1.211 | 1.209 | 1.209 |
| Evaluation of fitting property | | AA | AA | A | A | B | B | AA | AA |
| Evaluation of aesthetic property | | ○ | ○ | ○ | ○ | ○ | ○ | ○ | ○ |

EP 4 371 966 A1

| | | Example 9 | Example 10 | Example 11 | Example 12 | Example 13 | Comparative Example 1 | Comparative Example 2 | Comparative Example 3 |
|---|---|---|---|---|---|---|---|---|---|
| Stabilizer concentration | | 5.5 | 5.5 | 5.5 | 5.5 | 5.5 | 5.5 | 5.5 | 5.5 |
| Type of impregnating liquid | Solution (I) | Solution 1 | Solution 21 | Solution 24 | Solution 1 | Solution 30 | Solution 1 | Solution 1 | Solution 29 |
| | Solution (II) | Solution 2 | Solution 22 | Solution 25 | Solution 27 | Solution 31 | Solution 14 | Solution 16 | Solution 14 |
| | Solution (III) | Solution 3 | Solution 23 | Solution 26 | Solution 28 | Solution 32 | Solution 15 | Solution 17 | Solution 15 |
| Amount of impregnating liquid (g) | Solution (I) | 23.0 | 23.0 | 23.0 | 23.0 | 23.0 | 23.0 | 23.0 | 23.0 |
| | Solution (II) | 23.0 | 23.0 | 23.0 | 23.0 | 23.0 | 23.0 | 23.0 | 23.0 |
| | Solution (III) | 23.0 | 23.0 | 23.0 | 23.0 | 23.0 | 23.0 | 23.0 | 23.0 |
| Ctotal of impregnation liquid | Solution (I) | 8.180 | 8.980 | 8.800 | 8.180 | 8.180 | 8.180 | 8.180 | 4.480 |
| | Solution (II) | 7.260 | 8.260 | 8.670 | 7.470 | 7.260 | 0.260 | 2.96 | 0.260 |
| | Solution (III) | 7.360 | 8.360 | 8.730 | 7.530 | 7.360 | 0.360 | 3.06 | 0.360 |
| ΔCtotal | 含浸液 (I)-(II) | 0.920 | 0.720 | 0.130 | 0.710 | 0.920 | 7.920 | 5.220 | 4.220 |
| | 含浸液 (II)-(III) | 0.100 | 0.100 | 0.060 | 0.060 | 0.100 | 0.100 | 0.100 | 0.100 |
| | 含浸液 (I)-(III) | 0.820 | 0.620 | 0.070 | 0.650 | 0.820 | 7.820 | 5.120 | 4.120 |
| Supported element | Section A | Y, Pr, Nd | In, Pr, Nd | Y, Pr, Ga | Y, Pr, Nd | Y, Pr, Nd | Y, Pr, Nd | Y, Pr, Nd | Y, Pr, Nd |
| | Section B | Zr, Fe, Ni | Zr, Fe, Ni | Zr, Fe, Ga | Zr, Fe, At | Zr, Fe, Ni | Fe, Ni | Zr, Fe, Ni | Fe, Ni |
| Stabilizer concentration in zirconium compounds | | 0.0 | 0.0 | 0.0 | 0.0 | 0.0 | 0.0 | 0.0 | 0.0 |

17

(continued)

| | | Example 9 | Example 10 | Example 11 | Example 12 | Example 13 | Comparative Example 1 | Comparative Example 2 | Comparative Example 3 |
|---|---|---|---|---|---|---|---|---|---|
| Relative density | SectionA | 0.566 | 0.569 | 0.569 | 0.566 | 0.567 | 0.567 | 0.567 | 0.562 |
| | Section B | 0.565 | 0.567 | 0.565 | 0.565 | 0.565 | 0.556 | 0.559 | 0.556 |
| | Difference | 0.001 | 0.001 | 0.004 | 0.001 | 0.002 | 0.011 | 0.008 | 0.006 |
| Shrinkage rate | Enamel | 1.209 | 1.207 | 1.207 | 1.208 | 1.208 | 1.208 | 1.208 | 1.212 |
| | Central | 1.209 | 1.208 | 1.207 | 1.209 | 1.209 | 1.216 | 1.214 | 1.216 |
| | Servical | 1.210 | 1.208 | 1.207 | 1.209 | 1.209 | 1.216 | 1.214 | 1.216 |
| | Average | 1.209 | 1.208 | 1.207 | 1.209 | 1.209 | 1.213 | 1.212 | 1.215 |
| Evaluation of fitting property | | AA | AA | AA | AA | AA | C | C | B |
| Evaluation of aesthetic property | | ○ | ○ | ○ | ○ | ○ | ○ | ○ | x |

EP 4 371 966 A1

In Examples 1-2 and 7-13, because at least one of the impregnating solutions contains a zirconium component and the $\Delta C_{total}$ is 2.0 or less, the differences in the shrinkage rate between the enamel portion, the central portion and the servical portion are especially small, and especially good fitting property were exhbited.

In Examples 3-4, because at least one of the impregnating solutions contains a zirconium component and the $\Delta C_{total}$ is 4.0 or less, the differences in the shrinkage rate between the enamel portion, the central portion and the servical portion are small, and good fitting property were exhbited.

In Examples 5-6, because at least one of the impregnating solutions contains a zirconium component and the $\Delta C_{total}$ is 5.0 or less, the differences in the shrinkage rate between the enamel portion, the central portion and the servical portion are not remarkable, fitting property is in a degree that the prosthetics can be used by adjusting.

In Comparative Example 1, because a zirconium component is not contained in the impregnating solution and the $\Delta C_{total}$ exceeded 5.0, the differences in the shrinkage rate between the enamel portion, the central portion and the servical portion are large, therefore it is difficult to fit to the plaster model.

Although Comparative Example 2 contained a zirconium component in at least one of the impregnating solutions, because the $\Delta C_{total}$ exceeded 5.0, the differences in the shrinkage rate between the enamel portion, the central portion and the servical portion are large, therefore it is difficult to fit to the plaster model.

In Comparative Example 3, because no impregnation solutions contain a zirconium component, $C_{total}$ of each solution was less than 5.0 and therefore good translucency and good color tone were not obtained.

With respect to the use of substantially any plural and/or singular terms herein, those having skill in the art can translate from the plural to the singular and/or from the singular to the plural as is appropriate to the context.

Although the description herein has been given with reference to the drawings and embodiments, it should be noted that those skilled in the art may make various changes and modifications on the basis of this invention without difficulty. Accordingly, any such changes and modifications are intended to be included in the scope of the embodiments.

Industrial applicability

The present invention relates to a preparing method of a zirconia mill blank for dental cutting and machining having any one or more of translucency gradation, color gradation and strength gradation after perfect sintering by impregnating a zirconia calcined body with a plurality of impregnating solutions, which can suppress deterioration of the fitting property of a dental prosthesis device prepared from the zirconia mill blank for dental cutting and machining and a zirconia mill blank for dental cutting and machining having any one or more of translucency gradation, color gradation and strength gradation after perfect sintering and suppressing deterioration of the fitting property of a dental prosthesis device prepared from the zirconia mill blank for dental cutting and machining, and is a technology that can be used in the dental field.

**Claims**

1. A preparing method of a zirconia mill blank for dental cutting and machining having any one or more of translucency gradation, color gradation and strength gradation after perfect sintering, comprising

   a step of impregnating a zirconia calcined body with at least two types of impregnating solutions, wherein
   at least one of the impregnating liquids contains a zirconium component, and
   in a case in which the total of metal ion concentrations (mass%) contained in each impregnating liquid is defined as $C_{total}$,
   the difference $\Delta C_{total}$ which is a difference between $C_{total}$ of each impregnating liquid is 5.0 or less.

2. The preparing method according to claim 1, wherein
   the preparing method further comprises a step of drying the zirconia calcined body impregnated with the impregnating solution.

3. The preparing method according to claim 1 or 2, wherein
   the preparing method further comprises a step of degreasing the zirconia calcined body impregnated with the impregnating solution.

4. The preparing method according to any one of claims 1 to 3, wherein
   at least one of the impregnating solution contains a transition metal ion.

5. The preparing method according to any one of claims 1 to 3, wherein

at least one of the impregnating solution contains a rare earth metal ion.

6. The preparing method according to any one of claims 1 to 5, wherein
at least one of the impregnating solution contains any one or aluminum ion, gallium ion, and indium ion.

7. The preparing method according to any one of claims 1 to 6, wherein
the zirconium component in the impregnating solution is any one or more of zirconium oxychloride, zirconium oxyacetate, and zirconyl nitrate.

8. The preparing method according to any one of claims 1 to 7, wherein
at least one of the impregnating solution contains urea.

9. The preparing method according to any one of claims 1 to 8, wherein
at least one of the impregnating solution contains glycol.

10. The preparing method according to any one of claims 1 to 9, wherein
at least one of the impregnating solution contains a hydroxy acid.

11. The preparing method according to claim 10, wherein
the hydroxy acid is any one or more of citric acid, malic acid and lactic acid.

12. A zirconia mill blank for dental cutting and machining wherein

    in a case in which, in bewteen two opposing surfaces,

        a section from one end to 50% of a distance between the two opposing surfaces is defined as section A,
        a section from other end to 50% of the distance between the two opposing surfaces is defined as section B,

    the section A contains a region A in which a compound of a stabilizer element is supported in a pore,
    the section B contains a region B in which a zirconium compound is supported in a pore, and
    a concentration of a metal ion in the region A and a concentration of a metal ion in the region B are different and/or a type of a metal ion in the region A and a type of a metal ion in the region B are different.

13. The zirconia mill blank for dental cutting and machining comprisingaccoring to claim 12, wherein
a stabilizer concentration in the zirconium compound is lower than a stabilizer concentration in the zirconia mill blank for dental cutting and machining which constitutes a base material.

14. The zirconia mill blank for dental cutting and machining comprisingaccoring to claim 12 or 13, wherein
the stabilizer element is a rare earth metal.

15. The zirconia mill blank for dental cutting and machining comprisingaccoring to claim 14, wherein
the rare earth metal is yttrium.

16. The zirconia mill blank for dental cutting and machining comprisingaccoring to any one of claims 12 to 15, wherein
a compound of a coloring element is supported in the pore of at least one of the region A and the region B.

17. The zirconia mill blank for dental cutting and machining comprisingaccoring to claim 16, wherein
the coloring element is a rare earth metal.

18. The zirconia mill blank for dental cutting and machining comprisingaccoring to claim 16, wherein
the coloring element is a transition metal.

19. The zirconia mill blank for dental cutting and machining comprisingaccoring to any one of claims 12 to 18, wherein
at least one of an aluminum compound, a gallium compound and an indium compound is supported in the pore of at least one of the region A and the region B.

20. The zirconia mill blank for dental cutting and machining comprisingaccoring to any one of claims 12 to 19, wherein
at least one of a niobium compound and a tantalum compound is supported in the pore of at least one of the region A and the region B.

21. The zirconia mill blank for dental cutting and machining comprisingaccoring to any one of claims 12 to 20, wherein a difference in relative density between the region A and the region B is less than 0.008.

Europäisches
Patentamt

European
Patent Office

Office européen
des brevets

# EUROPEAN SEARCH REPORT

Application Number

EP 23 20 6599

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 3 892 254 A1 (SHOFU INC [JP]) 13 October 2021 (2021-10-13) * paragraphs [0011] – [0022], [0037], [0045]; claims 1-11; tables 1-3 * ----- | 6,8,10, 11,15,19 | INV. C04B41/00 C04B41/52 C04B41/89 C04B35/486 |
| X | EP 3 275 851 A1 (SHOFU INC [JP]) 31 January 2018 (2018-01-31) | 1-5,7, 12-14, 16-18, 20,21 | C04B35/632 C04B35/634 C04B35/638 A61C13/00 |
| Y | * paragraphs [0023] – [0024], [0034] – [0038]; examples 1-7 * ----- | 6,8-11, 15,19 | A61C13/083 A61K6/818 A61K6/822 |
| Y | EP 3 132 788 A1 (DENTAL DIREKT GMBH [DE]) 22 February 2017 (2017-02-22) * paragraphs [0025], [0033]; table 1 * ----- | 9 | ADD. C04B111/00 C04B111/82 |

TECHNICAL FIELDS
SEARCHED (IPC)

C04B
A61K
A61C

The present search report has been drawn up for all claims

| Place of search | Date of completion of the search | Examiner |
|---|---|---|
| Munich | 5 April 2024 | Bonneau, Sébastien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 20 6599

This annex lists the patent family members relating to the patent documents cited in the above-mentioned European search report.
The members are as contained in the European Patent Office EDP file on
The European Patent Office is in no way liable for these particulars which are merely given for the purpose of information.

05-04-2024

| Patent document cited in search report | | Publication date | Patent family member(s) | | Publication date |
|---|---|---|---|---|---|
| EP 3892254 | A1 | 13-10-2021 | CN | 113491591 A | 12-10-2021 |
| | | | EP | 3892254 A1 | 13-10-2021 |
| | | | JP | 2021165222 A | 14-10-2021 |
| | | | KR | 20210122703 A | 12-10-2021 |
| | | | US | 2021401553 A1 | 30-12-2021 |
| EP 3275851 | A1 | 31-01-2018 | EP | 3275851 A1 | 31-01-2018 |
| | | | EP | 4180406 A1 | 17-05-2023 |
| | | | JP | 6093900 B1 | 08-03-2017 |
| | | | JP | 2018015364 A | 01-02-2018 |
| | | | US | 2018028293 A1 | 01-02-2018 |
| EP 3132788 | A1 | 22-02-2017 | DE | 102015216056 A1 | 23-02-2017 |
| | | | EP | 3132788 A1 | 22-02-2017 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018015364 A


**Non-patent literature cited in the description**

- Lattice and Density for Y2O3-Stabilized ZrO2. *J. Am. Ceram. Soc.,* 1986, vol. 69 (4), 325-332